# EUROPEAN PATENT SPECIFICATION

(11) **EP 2 916 849 B1**
(45) Date of publication and mention of the grant of the patent: **18.01.2017**
(21) Application number: 13795189.3
(22) Date of filing: 31.10.2013
(51) Int. Cl.: A61K 31/728, A61K 36/185, A61K 36/23, A61K 36/48, A61K 36/758, A61P 15/02

(54) **COMPOSITIONS FOR VAGINAL USE CONTAINING TAMARIND POLYSACCHARIDES**
ZUSAMMENSETZUNGEN MIT TAMARINDPOLYSACCHARIDEN ZUR VAGINALEN VERWENDUNG
COMPOSITIONS À USAGE VAGINAL CONTENANT DES POLYSACCHARIDES DE TAMARIN

(30) Priority: 09.11.2012 IT MI20121917
(43) Date of publication of application: 16.09.2015
(73) Proprietor: Velleja Research SRL, 29010 Pontenure (PC) (IT)
(72) Inventor: DI PIERRO, Francesco, I-29010 Pontenure (PC) (IT)
(74) Representative: Bianchetti Bracco Minoja S.r.l.
(86) International application number: PCT/EP2013/072837
(87) International publication number: WO 2014/072230

(56) References cited:
- WO-A1-2007/054211
- WO-A1-2011/147767
- WO-A1-2011/147768
- WO-A2-2008/011556
- JP-A- 11 021 231
- MAIRE B. MAC BRIDE, MBBCH, DEBORAH J. RHODES, MD, AND LYNNE T. SHUSTER, MD: "Vulvovaginal Atrophy", MAYO CLIN PROC., vol. 85, no. 1, January 2010 (2010-01), pages 87-89, XP002692727,
- SHUKLA A ET AL: "In vitro and in vivo wound healing activity of asiaticoside isolated from", JOURNAL OF ETHNOPHARMACOLOGY, ELSEVIER SCIENTIFIC PUBLISHERS LTD, IE, vol. 65, no. 1, 21 July 1998 (1998-07-21), pages 1-11, XP028142720, ISSN: 0378-8741, DOI: 10.1016/S0378-8741(98)00141-X [retrieved on 2011-02-15]
- STEENKAMP V ET AL: "Studies on antibacterial, antioxidant and fibroblast growth stimulation of wound healing remedies from South Africa", JOURNAL OF ETHNOPHARMACOLOGY, ELSEVIER SCIENTIFIC PUBLISHERS LTD, IE, vol. 95, no. 2-3, 1 December 2004 (2004-12-01), pages 353-357, XP004990588, ISSN: 0378-8741, DOI: 10.1016/J.JEP.2004.08.020
- MOSHI M J ET AL: "Some pharmacological properties of extracts of Terminalia sericea roots", JOURNAL OF ETHNOPHARMACOLOGY, ELSEVIER SCIENTIFIC PUBLISHERS LTD, IE, vol. 97, no. 1, 10 February 2005 (2005-02-10), pages 43-47, XP027757194, ISSN: 0378-8741 [retrieved on 2005-02-10]
- DATABASE BIOSIS [Online] BIOSCIENCES INFORMATION SERVICE, PHILADELPHIA, PA, US; 2011, MARKOWSKA J ET AL: "The effect of hyaluronic acid (Cicatridine) on healing and regeneration of the uterine cervix and vagina and vulvar dystrophy therapy", XP002692726, Database accession no. PREV201100158699 & EUROPEAN JOURNAL OF GYNAECOLOGICAL ONCOLOGY, vol. 32, no. 1, 2011, pages 65-68, ISSN: 0392-2936
- PETRA M. CASEY, MD, MARGARET E. LONG, MD, AND MARY L. MARNACH, MD: "Abnormal Cervical Appearance: What to Do, When to Worry?", MAYO CLIN PROC., vol. 86, no. 2, February 2011 (2011-02), pages 147-151, XP002692728,

## Description

The present invention relates to vaginal compositions containing tamarind polysaccharides useful for the treatment of cervix ectropion.

### Background to the invention

Atrophic vaginitis is a disorder typical of the post-menopausal period, characterised by inflammation of the vaginal mucosa with a progressive reduction in the thickness of the vaginal epithelium.

After the onset of the menopause, the reduced secretion of steroidal hormones, in particular oestrogens, induces hypotrophy of the epithelium lining the vagina and consequent disappearance of the lining glycoproteins (glycogen), with a reduction in the thickness of the epithelium and a percentage reduction in the elastic component of the submucosa. This predisposes the sufferer to opportunistic infections (mild or moderate candidiasis), and produces a marked reduction in lubrication.

The treatment of this disorder consists of local and general administration of oestrogens. Local treatment usually involves administration of slow-release intravaginal ointments or pessaries. Systemic treatment involves oral administration of oestrogens.

The clinical signs and symptoms typical of atrophic vaginitis include dystrophy, dyspareunia, ectropion, vulvar itching, stinging and bleeding. In particular, cervical ectropion is an abnormal presence of intracervical mucosa, with simple single-layered columnar epithelium, in the area of the *portio vaginalis* that surrounds the external uterine orifice. This situation is often associated, in post-menopausal women, with "slipping" of said mucosa from the endocervical canal to the exterior. Its presence causes vaginal discharges (leucorrhoea), predisposes to vaginal infections (mainly bacterial vaginosis), and causes blood loss after sexual intercourse (spotting). The replacement of this single-layered epithelium with normal non-keratinised multilayered pavimentous epithelium can theoretically be promoted by the use of re-epithelialising/wound-healing preparations. Very often, however, they fail, or their success rate is modest, with the result that ablation by diathermocoagulation is required. In this case, the burnt tissue is replaced by normal lining tissue. However, the diathermocoagulation procedure may not last permanently, and relapses may follow in the subsequent months. There is therefore still a need for effective alternative treatments substantially devoid of side effects.

### Description of the invention

It has now been discovered that the use of a gel containing a water-ethanol extract of tamarind seeds counteracts the cervix ectropion diagnosed in physiologically post-menopausal women.

Tamarind seed extract is known and has mainly been proposed for use in ophthalmic formulations.

The vaginal gel formulations for the use to which the invention relates contain 0.5% to 5% by weight of tamarind seed extract, preferably 1 to 2% by weight.

The extract obtained from a water:ethanol mixture in the ratio of 20:70, characterised by a high presence of polysaccharides (assay > 50%) with a high molecular weight (> 100,000), is preferred.

The gel based on tamarind seed polysaccharides can contain other ingredients which give rise to an additive effect or add soothing/anti-inflammatory properties to the gel. Examples of said ingredients include polyphenol extracts, triterpenes obtained from *Centella asiatica* or *Terminalia sericea,* isobutylamides from *Zanthoxylum* spp, and hyaluronic acid.

The gel for use according to the invention can be prepared with conventional techniques and excipients, in particular using mucoadhesive agents such as cellulose derivatives (hydroxyethylcellulose and hydroxypropyl methylcellulose), polymers such as carbacol, colloids based on chitosan, and other substances commonly used as mucoadhesives for the vaginal area.

The gel must be processed in a water-cooled turboemulsifier at a temperature of around 20°C for at least 4 hours. Only after said procedure will the gel have the necessary consistency for its biological action. At higher temperatures, typically 30-40°C, used in turboemulsifiers for cream or gel preparations for gynaecological use, the mixture of tamarind polysaccharides is not sufficiently colloidal, and is clinically ineffective. At low temperatures, tamarind polysaccharides probably polymerise, forming macro-aggregates, and the resulting colloid is clinically effective in the treatment of ectropion.

Intravaginal administration for 30 days of 10 ml of a gel containing 1.0% tamarind seed polysaccharides thus manufactured reduced the diagnosis of ectropion in 75% of cases. Moreover, after a 120 day wash-out, only 5% of the women previously treated with the gel suffered a relapse. This value rose to 25% in women previously treated with a gel based on hyaluronate (1%). This treatment, administered for 30 days, reduced the diagnosis of ectropion by 25%.

The example below illustrates the invention in greater detail.

### Example

Gel for intravaginal use containing:

| | |
|---|---|
| Polysaccharides obtained from tamarind seeds | (1%) |
| Triterpenes from *Centella asiatica* | (1%) |
| Isobutylamides from *Zanthoxylum bungeanum* | (0.5%) |
| Sodium hyaluronan | (0.5%) |
| Noveon | (0.8%) |

## Claims

1. Vaginal compositions comprising an extract of tamarind seeds for use in the treatment and prevention of cervix ectropion.

2. Compositions for use according to claim 1 in gel form.

3. Compositions for use according to claim 1 or 2 wherein the extract is obtained by extraction of tamarind seeds with a water:ethanol mixture in the ratio of 20:70.

4. Compositions for use according to claim 2 wherein the extract has a polysaccharide content of 50%.

5. Compositions for use according to claim 4 wherein the polysaccharides have a molecular weight higher than 100,000.

6. Compositions for use according to one or more of claims 1 to 5 containing 0.5% to 5% by weight of tamarind seed extract, preferably 1 to 2% by weight.

7. Compositions for use according to one or more of claims 1 to 6 also containing polyphenol extracts, triterpenes from *Centella asiatica* or from *Terminalia sericea,* isobutylamides from *Zanthoxylum* spp, or hyaluronic acid, together with mucoadhesive agents.

## Patentansprüche

1. Vaginalzusammensetzungen, umfassend einen Extrakt aus Tamarindensamen für die Verwendung in der Behandlung und Vorbeugung von Zervixerosion.

2. Zusammensetzungen zur Verwendung gemäß Anspruch 1 in Gelform.

3. Zusammensetzungen zur Verwendung gemäß Anspruch 1 oder 2, worin der Extrakt erhalten wird durch Extraktion von Tamarindensamen mit einer Wasser/Ethanol-Mischung in dem Verhältnis 20 : 70.

4. Zusammensetzungen zur Verwendung gemäß Anspruch 2, worin der Extrakt einen Polysaccharidgehalt von 50 % aufweist.

5. Zusammensetzungen zur Verwendung gemäß Anspruch 4, worin die Polysaccharide ein Molekulargewicht von mehr als 100000 aufweisen.

6. Zusammensetzungen zur Verwendung gemäß einem der Ansprüche 1 bis 5, enthaltend 0.5 Gew.-% bis 5 Gew.-%, vorzugsweise 1 Gew.-% bis 2 Gew.-%, an Tamarindensamenextrakt.

7. Zusammensetzungen zur Verwendung gemäß einem der Ansprüche 1 bis 6, zusätzlich enthaltend Polyphenolextrakte, Triterpene von *Centella asiatica* oder von *Terminalia sericea,* Isobutylamide von *Zanthoxylum* spp oder Hyaluronsäure zusammen mit mucoadhesiven Mitteln.

## Revendications

1. Compositions vaginales comprenant un extrait de graines de tamarin pour leur utilisation dans le traitement et la prévention de l'ectropion du col de l'utérus.

2. Compositions pour leur utilisation selon la revendication 1, sous la forme de gels.

3. Compositions pour leur utilisation selon la revendication 1 ou 2, dans lesquelles on obtient l'extrait par extraction de graines de tamarin avec un mélange eau : éthanol dans le rapport 20 : 70.

4. Compositions pour leur utilisation selon la revendication 2, dans lesquelles l'extrait possède une teneur de 50 % en polysaccharides.

5. Compositions pour leur utilisation selon la revendication 4, dans lesquelles les polysaccharides possèdent un poids moléculaire supérieur à 100.000.

6. Compositions pour leur utilisation selon une ou plusieurs des revendications 1 à 5, contenant de 0,5 % à 5 % poids d'extrait de graines de tamarin, de préférence de 1 à 2 % poids.

7. Compositions pour leur utilisation selon une ou plusieurs des revendications 1 à 6, contenant également des extraits de polyphénol, des triterpènes issus de *Centella asiatica* ou de *Terminalia sericea,* des isobutylamides issus de *Zanthoxylum* spp., ou de l'acide hyaluronique, conjointement avec des agents mucoadhésifs.
